# EUROPEAN PATENT APPLICATION

(11) **EP 2 730 272 A2**
(43) Date of publication of application: **14.05.2014**
(21) Application number: 13192756.8
(22) Date of filing: 13.11.2013
(51) Int. Cl.: A61K 8/34, A61Q 19/00, A61K 8/891, A61K 8/894, A61K 8/895

(54) **Water-in-silicone oil macroemulsion cosmetic composition**

(30) Priority: 13.11.2012 JP 2012249300
(71) Applicant: Shin-Etsu Chemical Co., Ltd., Chiyoda-ku, Tokyo (JP)
(72) Inventor: Lee, Taeck-young, Seoul (KR)
(74) Representative: Stoner, Gerard Patrick

(57) **Abstract**

A water-in-silicone oil macroemulsion cosmetic composition of waterdrop quick break type is provided. The silicone oil phase part contains a partly crosslinked emulsifiable silicone elastomer, a partly crosslinked non-emulsifiable silicone elastomer, and silicone oil, and the aqueous phase part contains glycerin as a freeze stabilizer and optionally at least one member selected from polyols and lower alcohols and/or at least one member selected from organic acid salts and inorganic salts as a mixture of freeze stabilizers. The cosmetic composition instantaneously releases water upon application on the skin. The cosmetic composition is stable at the extremely low temperature of -20°C, and it retains its dispersion stability even after repeated freezing and thawing, and accordingly, and it can be used in a variety of cosmetic product.

## Description

### TECHNICAL FIELD

This invention relates to a water-in-silicone oil (W/S) macroemulsion cosmetic composition of waterdrop quick break type wherein the emulsion breaks instantaneously with the application of the cosmetic composition on the skin thereby releasing the water content.

### BACKGROUND

Cosmetic products are produced by using various emulsion systems (emulsion compositions) such as oil in water (O/W) emulsion, water in oil (W/O) emulsion, and water-in-silicone oil (W/S) emulsion to convey various components.

Cosmetic composition for skin care is directly applied to the skin, and therefore, the demands for such composition include not only the functionality but also good skin feeling. Recently, there is a growing interest for a sensuous cosmetic product which is capable of providing unique feeling during or after the use of the composition by the change of the emulsion system (emulsion composition) or the composition of the system.

An example is cool feel cosmetic composition having a menthol derivative added in the cosmetic composition (for example, Patent Document 1: Korean Patent No. 10-1084875). Another example is a cosmetic composition comprising a cationic copolymer containing monomer unit selected from acryloylethyltri(C₁-C₃ alkyl) ammonium salts and a mica having a carbon dioxide coating, and this cosmetic composition realizes a smooth skin by soft focus effect. (See for example, Patent Document 2: WO 2009/103602.)

Another type of products that are in focus is waterdrop quick break type products which intensively supply water component to the skin simultaneously with the application of the product to the skin by bursting of the water droplets.

Also disclosed is a cosmetic product providing the skin with a moist feeling like that of the lotion as well as cool feeling and humectant feeling, and which has also realized excellent make up effects and stability. This composition is prepared by mixing PEG/PPG-19/19 dimethicone or lauryl PEG-9 polydimethylsiloxyethyl dimethicone with the oil phase of the water-in-oil emulsion composition, and when applied to the skin, the interior aqueous phase is instantaneously released simultaneously with the breakage of the emulsion of the water-in-oil foundation emulsion. (See for example, Patent Document 3: Korean Patent No. 10-1158281.)

Water-in-silicone and silicone-in-water emulsion compositions having excellent waterdrop quick break effect, cosmetic retention, skin adhesion, and skin feeling are also proposed. The emulsion composition comprises a polyionic complex of an anionic hydrophilic macromolecule substance bonded with a hydrophobic cationic monomer by ionic bond, a silicone based crosspolymer, and a silicone oil. (See for example, Patent Document 4: Korean Patent No. 10-1070819.)

As disclosed in the Patent Document 3, the emulsion composition should be highly stable at various temperatures if the emulsion composition is to be used in a cosmetic product.

Stability of the cosmetic composition is generally evaluated in a cyclic evaluation by observing the dispersion state of the emulsion at different temperatures (high temperature, low temperature, and room temperature) and in different atmospheres. Patent Document 3 discloses an observation of the dispersion state by repeating the incremental 1 month cycle of 45°C → room temperature →5°C. In this case, the observation is conducted by occasionally changing the temperature and time depending on the dosage form of the cosmetic product.

The use of 5°C for the "low" temperature may be adequate in the case of the indoor use. The cosmetic product, however, may be exposed to a lower temperature in the delivery and storage of the cosmetic product, and also, in the use of the product in colder district, and at a temperature lower than 5°C, there is a risk that the emulsion is destroyed inviting separation of the aqueous phase part and the oil phase part.

Accordingly, the product is also tested by a freeze test at a temperature of lower than -20°C, and also, by a repeated freeze-thaw stability test cycles as a preparation for shipping to cold district.

### Citation List

Patent Document 1: Korean Patent No. 10-1084875
Patent Document 2: WO 2009/103602
Patent Document 3: Korean Patent No. 10-1158281
Patent Document 4: Korean Patent No. 10-1070819

### SUMMARY OF INVENTION

### Technical Problem

An object of the present invention is to provide a water-in-silicone oil (W/S) macroemulsion cosmetic composition which can be produced into a waterdrop quick break type product which intensively supply water component to the skin simultaneously with the application of the product to the skin by bursting of the waterdrop, and which is capable of retaining its stability in repeated freeze-thaw cycles.

### Solution to Problem

The inventors of the present invention made an intensive study to realize the object as described above, and found that a waterdrop quick break-type preparation can be produced by using a constitution comprising a silicone oil phase part (S) comprising a partly crosslinked emulsifiable silicone elastomer, a partly crosslinked non-emulsifiable silicone elastomer, a non-crosslinked silicone emulsifier, and a silicone oil, and an aqueous phase part (W) comprising glycerin at an amount of 3 to 15% by weight of the entire composition as a freeze stabilizer. The inventors also found that a water-in-silicone oil macroemulsion cosmetic product having an improved freeze and thaw stability can be obtained by using such constitution. The present invention has been completed on the bases of such finding.

Accordingly, the present invention provides a water-in-silicone oil (W/S) macroemulsion cosmetic composition comprising a silicone oil phase part (S) and an aqueous phase part (W) as described below.
[1] A water-in-silicone oil macroemulsion cosmetic composition comprising a silicone oil phase part and an aqueous phase part, wherein
   the silicone oil phase part comprises a partly crosslinked emulsifiable silicone elastomer, a partly crosslinked non-emulsifiable silicone elastomer, a non-crosslinked silicone emulsifier, and a silicone oil, and
   the aqueous phase part contains glycerin at an amount of 3 to 15% by weight of the entire composition. The glycerine acts as a freeze stabilizer.
[2] A water-in-silicone oil macroemulsion cosmetic composition according to [1] further comprising at least one member selected from polyols and lower alcohols as a freeze stabilizer in the aqueous phase part at an amount of 3 to 15% by weight of the entire composition.
[3] A water-in-silicone oil macroemulsion cosmetic composition according to [2] wherein the polyol is 1,3-butylane glycol.
[4] A water-in-silicone oil macroemulsion cosmetic composition according to [2] or [3] wherein the lower alcohol is at least one member selected from methanol, ethanol, isopropanol, butanol, pentanol, and combinations thereof.
[5] A water-in-silicone oil macroemulsion cosmetic composition according to any one of [1] to [4] further comprising at least one member selected from organic acid salts and inorganic salts as the freeze stabilizer in the aqueous phase part at an amount of 0.4 to 1.5% by weight of the entire composition.
[6] A water-in-silicone oil macroemulsion cosmetic composition according to [5] wherein the organic acid salt is at least one member selected from the group consisting of sodium citrate, sodium formate, sodium acetate, and potassium acetate.
[7] A water-in-silicone oil macroemulsion cosmetic composition according to [5] or [6] wherein the inorganic salt is at least one member selected from sodium chloride, potassium chloride, calcium chloride, magnesium chloride, and magnesium sulfate.

### A method of making such compositions is a further aspect.

### Advantageous Effects

The water-in-silicone oil (W/S) macroemulsion cosmetic composition of the present invention will attract users since it has enabled visual confirmation of the water supply to the skin by instantaneous water release. The water-in-silicone oil macroemulsion cosmetic compositions described herein have also been found stable at an extremely low temperature of -20°C, and the cosmetic composition is capable of retaining the stable condition after repeated freeze and thaw cycles. Accordingly, the cosmetic composition can be used in various cosmetic product.

### BRIEF DESCRIPTION OF DRAWINGS

FIG. 1 is a picture taken by an optical microscope of the particles of the water-in-silicone oil (W/S) macroemulsion produced in Example 1.
FIGS. 2(a) to 2(c) are respectively pictures taken by an optical microscope of the water-in-silicone oil (W/S) macroemulsions produced in Examples 8 and 9 and Comparative Example 4.

### FURTHER EXPLANATIONS; OPTIONS AND PREFERENCES

Next, the present invention is described in detail.

The present invention provides a water-in-silicone oil (W/S) macroemulsion cosmetic composition which can be applied for a cosmetic product of waterdrop quick break-type which instantaneously releases water upon its application on the skin by the breakage of the emulsion. The water-in-silicone oil (W/S) macroemulsion cosmetic composition comprises the aqueous phase part (W) and the silicone oil phase part (S), and the aqueous phase part (W) contains a freeze stabilizer in the water to thereby enable retention of the stable dispersion phase after repeated freeze and thaw cycles.

The freeze stabilizer used is glycerin, and the content in relation to the entire composition is limited to realize the stability.

The glycerin is used at 3 to 15% by weight, and preferably at 5 to 10% by weight of the entire cosmetic composition. The content below such range may invite phase separation in the repeated cycles of freezing and thawing or during the storage at an extremely low temperature of -20°C. The content beyond such range may be economically disadvantageous in view of the slight improvement in the advantageous effects, and also, in view of sticky feeling during its use, and therefore, the content is preferably in the range as described above.

The cosmetic may further contain at least one member selected from polyols and lower alcohols at an amount in relation to the entire composition of 3 to 15% by weight in the aqueous phase part (W) as the freeze stabilizer of the present invention in addition to the glycerin.

The polyol used may be 1,3-butylene glycol.

The lower alcohol is preferably a lower alcohol selected from methanol, ethanol, isopropanol, butanol, pentanol, and combinations thereof, and such lower alcohol may function as an agent for lowering the freezing point of the emulsion, and as a consequence, the freeze-thaw stability is improved.

The aqueous phase part (W) of the water-in-silicone oil (W/S) macroemulsion cosmetic composition, may further comprise at least one member selected from organic acid salts and inorganic salts capable of lowering the freezing point at a content of 0.4 to 1.5% by weight in the cosmetic composition as the freeze stabilizer to thereby improve the freeze and thaw stability.

The organic acid salt used may be sodium citrate, sodium formate, sodium acetate, potassium acetate, or the like, and the inorganic salt used may be the one selected from the group consisting of sodium chloride, potassium chloride, calcium chloride, magnesium chloride, magnesium sulfate, and combinations thereof.

The aqueous phase part (W) may contain glycerin as the freeze stabilizer as described above, or alternatively, at least one member selected from polyols and lower alcohols, and also, at least one member selected from organic acid salts and inorganic salts as the freeze stabilizer in addition to the glycerin. The water used in the aqueous phase part is preferably purified water.

The freeze stabilizer may be used in various emulsion compositions, and preferably, the freeze stabilizer is used in an emulsion cosmetic composition for producing the cosmetic product of waterdrop quick break type described in the present invention which instantaneously releases the water content by breakage of the emulsion when the cosmetic product is applied to the skin.

The emulsion cosmetic composition according to the present invention is a water-in-silicone oil (W/S) emulsion comprising an aqueous phase part (W) and a silicone oil phase part (S) mainly comprising a silicone oil agent. It is to be noted that the silicone oil phase part (S) may contain a non-silicone hydrocarbon oil agent at a content of typically up to 50% by weight in relation to the entire silicone oil phase part (S).

The silicone oil phase part (S) mainly comprising a silicone oil agent may further comprise a partly crosslinked emulsifiable silicone elastomer, a partly crosslinked non-emulsifiable silicone elastomer, and a non-crosslinked silicone emulsifier, and also, commonly used additives (for example, non-silicone hydrocarbon oil agent) in the silicone oil.

The silicone oil in the silicone oil phase part (S) is not particularly limited in the present invention as long as it is a non-crosslinked and non-reactive silicone oil, and any known silicone oils may be used. Examples include dimethicone, diphenyl dimethicone, diphenylsiloxyphenyl trimethicone, a mixture of dimethicone and cyclopentasiloxane, and cyclopentasiloxane.

Exemplary non-limiting commercially available silicone oils include KF-96A series, KF-96 series, KF-53, KF-54, KF-56A, KF-9008, KF-9011, KF-9014, X-21-5495, KF-9028, KF-995, and the like manufactured by Shin-Etsu Chemical Co., Ltd., and the preferred is dimethicone (KF-96A-6cs).

The silicone oil is typically incorporated at an amount of approximately 25 to 85% by weight in relation to the total weight of the silicone oil phase part (S).

The partly crosslinked emulsifiable silicone elastomer is not particularly limited in the present invention, and any silicone oil known in the art may be used. Examples include a mixture of dimethicone/PEG-10/15 crosspolymer and dimethicone, a mixture of dimethicone/PEG-10/15 crosspolymer and cyclopentasiloxane, a mixture of PEG-15/lauryl dimethicone crosspolymer and a mineral oil, a mixture of PEG-15/lauryl dimethicone crosspolymer and isododecane, a mixture of PEG-15/lauryl dimethicone crosspolymer and triethylhexanoi, a mixture of PEG-10/lauryl dimethicone crosspolymer and PEG-15/lauryl dimethicone crosspolymer and squalane, a mixture of PEG-15/lauryl polydimethylsiloxyethyl dimethicone crosspolymer and isododecane, a mixture of PEG-15/lauryl polydimethylsiloxyethyl dimethicone crosspolymer and cyclopentasiloxane, a mixture of dimethicone/polyglycerin-3 crosspolymer and dimethicone, a mixture of lauryl dimethicone/polyglycerin-3 crosspolymer and a mineral oil, a mixture of lauryl dimethicone/polyglycerin-3 crosspolymer and isododecane, a mixture of lauryl dimethicone/polyglycerin-3 crosspolymer and triethylhexanoin, a mixture of lauryl dimethicone/polyglycerin-3 crosspolymer and squalane, a mixture of polyglycerin-3/laurylpolydimethylsiloxyethyl dimethicone crosspolymer and isododecane, and a mixture of polyglycerin-3/laurylpolydimethylsiloxyethyl dimethicone crosspolymer and cyclopentasiloxane.

Exemplary non-limiting commercially available partly crosslinked emulsifiable silicone elastomers include KSG-210, KSG-240, KSG-310, KSG-320, KSG-330, KSG-340, KSG-320Z, KSG-350Z, KSG-710, KSG-810, KSG-820, KSG-830, KSG-840, KSG-820Z, KSG-850Z, and the like manufactured by Shin-Etsu Chemical Co., Ltd., and the preferred is KSG-210 (a mixture of dimethicone/PEG-10/15 crosspolymer and dimethicone).

The partly crosslinked emulsifiable silicone elastomer is typically incorporated at an amount of approximately 3 to 50% by weight in relation to the total weight of the silicone oil phase part (S).

The partly crosslinked non-emulsifiable silicone elastomer is not particularly limited in the present invention, and any silicone oil known in the art may be used. An example is a mixture of a silicone crosspolymer and at least one hydrocarbon oil and/or one silicone oil.

Examples include a mixture of dimethicone/vinyl dimethicone crosspolymer and cyclopentasiloxane, a mixture of dimethicone/vinyl dimethicone crosspolymer and dimethicone, a mixture of dimethicone/vinyl dimethicone crosspolymer and methyl triethicone, a mixture of dimethicone/vinyl dimethicone crosspolymer and isododecane, a mixture of dimethicone/phenylvinyl dimethicone crosspolymer and diphenylethylsiloxyphenyl trimethicone, a mixture of vinyl dimethicone/lauryl dimethicone crosspolymer and a mineral oil, a mixture of vinyl dimethicone/lauryl dimethicone crosspolymer and isododecane, a mixture of vinyl dimethicone/lauryl dimethicone crosspolymer and triethylhexanoin, a mixture of vinyl dimethicone/lauryl dimethicone crosspolymer and squalane, a mixture of laurylpolydimethylsiloxyethyl dimethicone/bis-vinyl dimethicone crosspolymer and isododecane, and a mixture of laurylpolydimethylsiloxy ethyl dimethicone/bis-vinyl dimethicone crosspolymer and cyclopentasiloxane.

Exemplary non-limiting commercially available partly crosslinked non-emulsifiable silicone elastomers include KSG-15, KSG-16, KSG-1610, KSG-106, KSG-18A, KSG-41, KSG-42, KSG-43, KSG-44, KSG-042Z, KSG-045Z, and the like manufactured by Shin-Etsu Chemical Co., Ltd., and the preferred is KSG-15 (a mixture of dimethicone/vinyl dimethicone crosspolymer and cyclopentasiloxane).

The partly crosslinked non-emulsifiable silicone elastomer is typically incorporated at an amount of approximately 2 to 50% by weight in relation to the total weight of the silicone oil phase part (S).

It is to be noted that the silicone oil, the partly crosslinked emulsifiable silicone elastomer, and the partly crosslinked non-emulsifiable silicone elastomer may be used so that the partly crosslinked emulsifiable silicone elastomer is typically at 0.05 to 1.5 parts by weight, and the partly crosslinked non-emulsifiable silicone elastomer is typically at 0.025 to 1.5 parts by weight in relation to 1 part by weight of the silicone oil.

The non-crosslinked silicone emulsifier is not particularly limited in the present invention, and any silicone oil known in the art may be used. Examples in clued polyether modified silicone or polyglycerin modified silicone such as PEG-3 dimethicone, PEG-10 dimethicone, PEG-9 methyl ether dimethicone, PEG-9 polydimethylsiloxyethyl dimethicone, lauryl PEG-9 polydimethylsiloxyethyl dimethicone, polyglyceryl-3 polydimethylsiloxyethyl dimethicone, and lauryl polyglyceryl-3 polydimethylsiloxyethyl dimethicone.

Exemplary non-limiting commercially available non-crosslinked silicone emulsifiers include KF-6015, KF-6016, KF-6017, KF-6028, KF-6038, KF-6104, KF-6105, and the like manufactured by Shin-Etsu Chemical Co., Ltd., and the preferred is KF-6017 (PEG-10 dimethicone).

The non-crosslinked silicone emulsifier is typically incorporated at an amount of approximately 0.03 to 11.5% by weight in relation to the total weight of the silicone oil phase part (S).

It is to be noted that, in the present invention, all of the silicone oil, the partly crosslinked emulsifiable silicone elastomer, the partly crosslinked non-emulsifiable silicone elastomer, and the non-crosslinked silicone emulsifier are preferably those having a cone penetration (worked penetration) at 25°C of approximately 300 to 450, and in particular, approximately 330 to 430. The cone penetration (worked penetration) may be measured in accordance with JIS K2220.

More specifically, a water-in-silicone oil (W/S) macroemulsion cosmetic composition in a specific aspect of the present proposals comprises
a) an aqueous phase part (W) comprising
   i) 3 to 15% by weight of glycerin, and
   ii) the remainder of water, and
b) a silicone oil phase part (S) comprising
   i) 1.0 to 15% by weight of partly crosslinked emulsifiable silicone elastomer,
   ii) 0.5 to 10% by weight of partly crosslinked non-emulsifiable silicone elastomer,
   iii) 0.01 to 5.0% by weight of non-crosslinked silicone elastomer, and
   iv) 5 to 30% by weight of silicone oil, with the proviso that entire composition (sum of the content of each component) is 100% by weight.

When the composition further comprises a polyol and/or a lower alcohol, a water-in-silicone oil (W/S) macroemulsion cosmetic composition in a specific aspect of the present proposals comprises
a) an aqueous phase part (W) comprising
   i) 3 to 15% by weight of glycerin, and
   ii) 5 to 15% by weight of at least one member of polyol and/or lower alcohol,
   iii) the remainder of water, and
b) a silicone oil phase part (S) comprising
   i) 1.0 to 15% by weight of partly crosslinked emulsifiable silicone elastomer,
   ii) 0.5 to 10% by weight of partly crosslinked non-emulsifiable silicone elastomer,
   iii) 0.01 to 5.0% by weight of non-crosslinked silicone elastomer, and
   iv) 5 to 30% by weight of silicone oil, with the proviso that entire composition (sum of the content of each component) is 100% by weight.

When the composition further comprises an organic acid salt and/or an inorganic salt, a water-in-silicone oil (W/S) macroemulsion cosmetic composition in a specific aspect of the present proposals comprises
a) an aqueous phase part (W) comprising
   i) 3 to 15% by weight of glycerin, and
   ii) 0.4 to 1.5% by weight of at least one member of organic acid salt and inorganic salt,
   iii) the remainder of water, and
b) a silicone oil phase part (S) comprising
   i) 1.0 to 15% by weight of partly crosslinked emulsifiable silicone elastomer,
   ii) 0.5 to 10% by weight of partly crosslinked non-emulsifiable silicone elastomer,
   iii) 0.01 to 5.0% by weight of non-crosslinked silicone elastomer, and
   iv) 5 to 30% by weight of silicone oil; or
a) an aqueous phase part (W) comprising
   i) 3 to 15% by weight of glycerin, and
   ii) 5 to 15% by weight of at least one member of polyol and/or lower alcohol,
   iii) 0.4 to 1.5% by weight of at least one member of organic acid salt and inorganic salt,
   iv) the remainder of water, and
b) a silicone oil phase part (S) comprising
   i) 1.0 to 15% by weight of partly crosslinked emulsifiable silicone elastomer,
   ii) 0.5 to 10% by weight of partly crosslinked non-emulsifiable silicone elastomer,
   iii) 0.01 to 5.0% by weight of non-crosslinked silicone elastomer, and
   iv) 5 to 30% by weight of silicone oil, with the proviso that entire composition (sum of the content of each component) is 100% by weight.

The production of the water-in-silicone oil (W/S) macroemulsion cosmetic composition having the composition as described above may be accomplished by applying the production schemes commonly used for the production of cosmetic compositions.

If desired, the cosmetic composition may also contain water soluble physiologically active component, oil soluble physiologically active component, solvent, additive, or the like which is known in the art. The solvent is the one used for dissolving the oil-soluble components, and a solvent such as ethanol may be used for this purpose.

Exemplary additives include humectant, fatty acid, antiseptic, pH adjusting agent, antioxidant, UV filter, pigment, dye, flavor, stabilizer, and thickener, which may be adequately selected by those skilled in the art.

The humectant may be one member selected from the group consisting of erythritol, xylitol, maltitol, propylene glycol, sorbitol, polyglycerin, polyethylene glycol, 1,2-pentanediol, isopropylene glycol, amino acid, sodium lactate, sodium pyrrolidonecarboxylate, xyloglucan, quince seed, carageenan, pectin, mannan, curdlan, galactan, dermatan sulfate, glycogen, keratan sulfate, chondroitin, mucoitinsulfuric acid, keratosulfate, locust bean gum, succinoglycan, calonym acid, hyaluronic acid, heparin sulfate, sodium hyaluronate, collagen, mucopolysaccharide, chondroitin sulfate, dimethylpolysiloxane, methylphenylsiloxane, supernatant of lactobacillus or Bifidobacterium, and mixtures thereof.

Exemplary fatty acids include one fatty acid selected from the group consisting of lauric acid, myristic acid, palmitic acid, stearic acid, oleic acid, linolenic acid, and mixtures thereof.

The thickener is used for the purpose of providing adequate viscosity with the cosmetic composition during its use to thereby improve feeling of the use. The thickener may be one member selected from the group consisting of sodium alginate, xanthan gum, aluminum silicate, quince seed extract, gum arabic, hydroxyethyl guar gum, carboxymethyl guar gum, guar gum, dextran, tragacanth gum, cellulose, hydroxypropyl cellulose, methyl hydroxypropyl cellulose, methylcellulose, carboxymethylcellulose, hydroxyethylcellulose, carboxyvinyl polymer, polyvinyl alcohol, polyvinylpyrrolione, and combinations thereof. Preferably, the thickner used is a carbomer which is capable functioning as a thickner at a wide range of pH even and at a thin concentration, with the transparency retained.

The lipid used may be at least one member selected from the group consisting of mango butter, shea butter, cocoa seed butter, macadamia nut oil, batyl alcohol, behenyl alcohol, cetostearyl alcohol, cetyl alcohol, stearyl alcohol, and combinations thereof.

Examples of the antiseptic used include benzoate salt, salicylate salt, sorbate salt, dihydroactate salt, paraoxybenzoate ester, 2,4,4-trichloro-2-hydroxydiphenyl ether, 3,3,4-trichlorocarbanilide, benzalkonium chloride, hinokitiol, and resorcin.

Examples of the pH adjusting agent used include sodium hydroxide, triethanolamine, citric acid, sodium citrate, boric acid, borax, and potassium hydrogen phosphate.

Examples of the antioxidant used include dibutylhydroxytoluene, butylhydroxyanisole, propyl gallate, and ascorbic acid.

Examples of the UV filter used include p-aminobenzoic acid UV absorbers,
anthranilic acid UV absorbers, salicylic acid UV absorbers, cinnamic acid UV absorbers, benzophenone UV absorbers, suger UV absorbers, 3-(4-methylbenzylidene)-d-camphor, 3-benzylidene-d,l-camphor, urocanic acid, ethyl urocanate ester, 2-phenyl-5-methylbenzoxazole, 2,2-hydroxy-5-methylphenylbenzotriazole, 2-(2-hydroxy-5-t-octylphenyl)benzotriazole, 2-(2-hydroxy-5-methylphenyl)benzotriazole, dibenzalazine, dianisoylmethane, 4-methoxy-4-t-butyl benzoyl methane, 5-(3,3-dimethyl-2-norbornylidene)-3-pentan-2-one, 2-hydroxy-4-methoxybenzophenone, octyldimethyl p-aminobenzoate, ethylhexyl p-methoxy cinnamate, titanium oxide fine particles, and zinc oxide fine particles.

Examples of the pigment used include silica, mica, talc, sericite, barium sulfate, titanium dioxide, chromium oxide, iron oxide, zinc oxide, cerium dioxide, zirconium dioxide, carbon black, barium, strontium, nylon powder, and polymethyl methacrylate (PMMA).

Examples of the antiseptic used include parabens such as methyl paraben and propyl paraben, phenoxyethanol, octanediol, and hexanediol.

With regard to the optional components as described above, a water-soluble component is preferably added to the aqueous phase, and oil phase component is preferably added to the silicone oil phase.

The method for producing the water-in-silicone oil macroemulsion of the present invention is not particularly limited, and any method used for emulsification in the art may be used for the production.

More specifically, the method for producing the water-in-silicone oil macroemulsion of the present invention comprises the steps of
S1) mixing a partly crosslinked emulsifiable silicone elastomer, a partly crosslinked non-emulsifiable silicone elastomer, and a non-crosslinked silicone emulsifier with a silicone oil to produce the silicone oil phase,
S2) mixing glycerin as a freeze stabilizer, and optionally, at least one freeze stabilizer selected from polyols and lower alcohols and/or at least one freeze stabilizer selected from organic acid salts and inorganic salts with water to produce the aqueous phase, and
S3) adding the aqueous phase part to the silicone oil phase part to produce the water-in-silicone oil (W/S) macroemulsion.

In this case, the ratio of the silicone oil phase part to the aqueous phase part is adequately selected. The weight ratio of the silicone oil phase part (S): the aqueous phase part is preferably 7:93 to 40:60 in view of simultaneously satisfying the requirements for the stability of the cosmetic composition and good feeling during the use.

Excessively high proportion of the silicone oil phase part may result in the loss of waterly feel in the use of the cosmetic composition while excessively high proportion of the aqueous phase part may result in the loss of the stability of the cosmetic composition.

Next, the production method is described in detail step by step.

First, the silicone oil component, the crosslinked emulsifiable silicone elastomer, the partly crosslinked non-emulsifiable silicone elastomer, the non-crosslinked silicone emulsifier, and other optional additives are mixed to prepare the silicone oil phase part (S). (S1)

The production of the silicone oil phase part may be conducted by the method commonly used in the art in an agitatable and temperature-regulatable vacuum emulsification tank with optional agitationa and heating to homogeneously mix other components in the silicone oil.

If necessary, an oil-soluble physiologically active component may also be added in addition to other additives in this stage, and exemplary oil-soluble physiologically active components used include any of those known in the field, for example, active component having wrinkle improving effects such as retinol and derivatives thereof (for example, retinol palmitate), adenosine, and the like.

Next, glycerin, and if necessary, at least one member selected from polyol and lower alcohol and/or at least one member selected from organic acid salt and inorganic salt are added to the water to thereby produce the aqueous phase part (W).

As in the case of production of the silicone oil phase part, the production of the aqueous phase part (W) may be conducted by the method commonly used in the art in an agitatable and temperature-regulatable vacuum emulsification tank with optional agitation and heating to homogeneously mix other components in the water.

If necessary, a water-soluble physiologically active component may also added in this stage, and exemplary water-soluble physiologically active components used include any of those known in the field, for example, active component having whitening effects such as oil-soluble licorice, ethylascorbyl ether, α-bisabolol, and the like.

Next, the aqueous phase part as described above is added to the silicone oil phase part to produce the water-in-silicone oil (W/S) macroemulsion. (S3)

The water-in-silicone oil macroemulsion may be produced in a vacuum emulsifying tank by agitating the mixture with a dispersion mixer at a speed of 1,000 to 3,000 rpm, and preferably 1,200 to 2,500 rpm for 1 to 10 minutes.

The thus produced water-in-silicone oil macroemulsion is preferably produced in the form of an emulsion having a particle size larger than the ordinary emulsion particles, namely, a particle size with the average size of 5 to 20 µm. The water-in-silicone oil macroemulsion having the particle size of such range is produced in the particle size larger than the conventional fine emulsion particles, and when brought in contact with the skin or a pressure is applied, breakage of the emulsion instantaneously results in the release of water content (namely, the aqueous phase portion) with the relative increase in the contact area with the skin, and hence, increase in the percutaneous absorption and percutaneous absorption area.

More specifically, the water-in-silicone oil macroemulsion of the present invention can retain stable dispersion phase at a high temperature, room temperature, and low temperature, and the retention of the stable dispersion phase was confirmed after repeated freezing and thawing at -20°C and room temperature.

Such water-in-silicone oil macroemulsion can be used in various cosmetic composition of various dosage form including solution, suspension, milky lotion, paste, gel, cream, lotion, powder, cleasing cream, oil, foundation, spray, and the like.

For example, the water-in-silicone oil macroemulsion composition may be used as a composition of basic cosmetics selected from moisturizer, nutrient lotion, lotion, cream, pack, gel, patch, and spray (mist) as well as composition of color tone cosmetics selected from lip stick, make up base, and foundation.

The composition of each dosage form may contain various matrix and additives required and adequate for the production of the preparation of the dosage form, and the composition may known compounds such as nonionic surfactant, silicone polymer, extender, flavor, antiseptic, bactericide, oxidation stabilizer, organic solvent, ionic or nonionic thickener, softener, antioxidant, free radical breaker, opacification agent, stabilizer, emollient, silicone, α-hydroxy acid, antifoaming agent, humectant, vitamin, insect repellent, flavor, preservative, surfactant, antiphlogistic, substance P antagonist, filler, polymer, propellant, basification or acidification agent, or colorant to the extent not adversely affecting the merits of the composition.

### EXAMPLES

Next, the present invention is described in further detail by referring to Examples which by no means limit scope of the invention since various alteration and modification can be made on the present invention. The Examples of the present invention are provided to more fully explain the present invention to those skilled in the art.

### Examples and Comparative Examples

### Production of emulsion compositions

Water-in-silicone oil macroemulsion compositions having the composition as shown in the following Tables 1 to 3 were produced.

First, the constituents of the silicone oil phase part were introduced in a vessel, and the mixture was agitated in a dispersion mixer at 1,000 rpm for 3 minutes to prepare the homogeneous oil phase part mixture. Next, the constituents of the aqueous phase part were introduced in a vessel, and the mixture was agitated in a dispersion mixer at 1,000 rpm for 3 minutes to prepare the aqueous phase part. This aqueous phase part was added to the silicone oil phase part, and the mixture was agitated at 1,500 rpm for 5 minutes to produce a water-in-silicone oil macroemulsion composition.

The partly crosslinked emulsifiable silicone elastomer ("emulsifiable elastomer") used was a mixture of dimethicone/PEG-10/15 crosspolymer and dimethicone (manufactured by Shin-Etsu Chemical Co., Ltd., KSG-210), the partly crosslinked non-emulsifiable silicone elastomer ("non-emulsifiable elastomer") was a mixture of dimethicone/vinyl dimethicone crosspolymer and cyclopentasiloxane (manufactured by Shin-Etsu Chemical Co., Ltd., KSG-15), the non-crosslinked silicone emulsifier was PEG-10 dimethicone (manufactured by Shin-Etsu Chemical Co., Ltd., KF-6017), and the silicone oil was dimethicone.

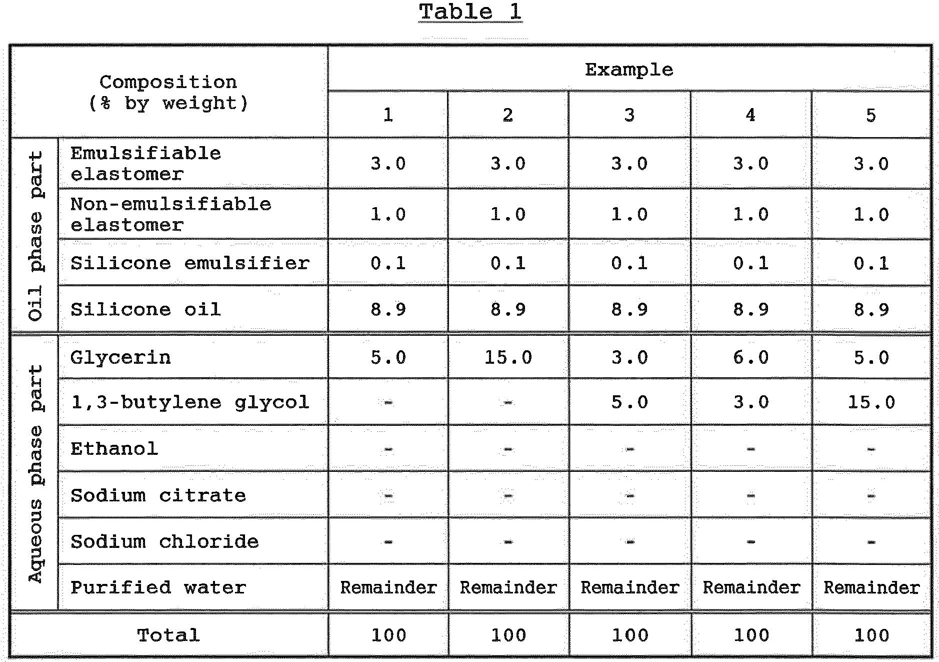

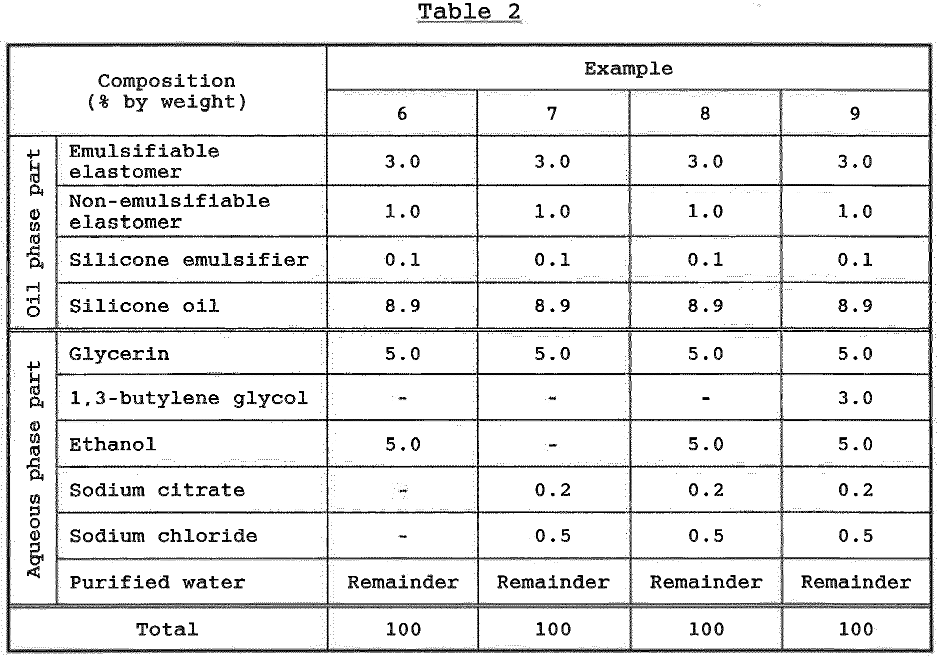

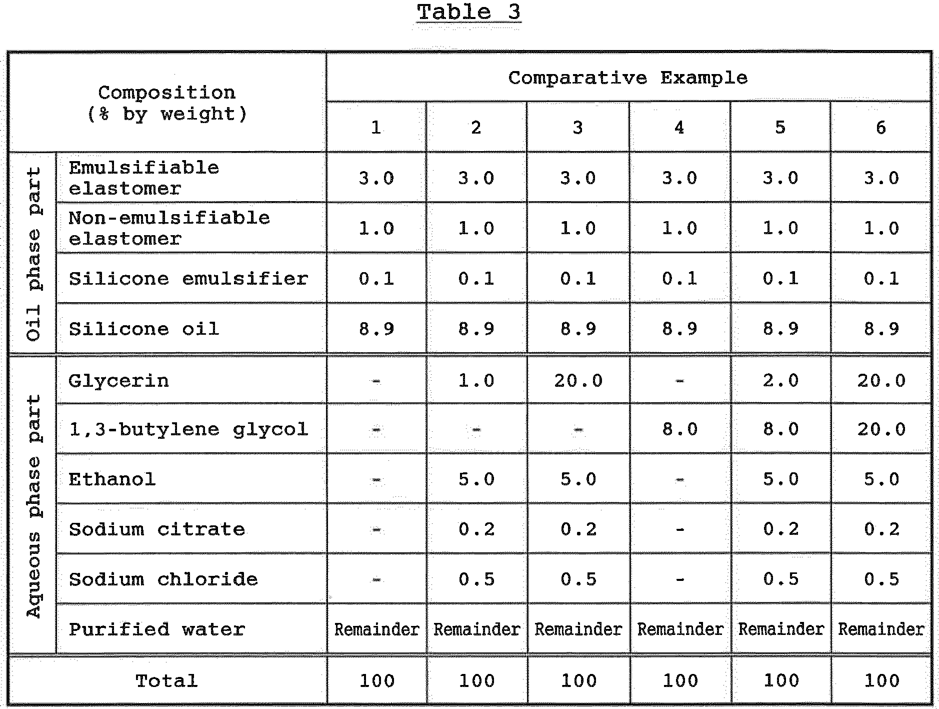

### Test Example 1

### Particles size and distribution state

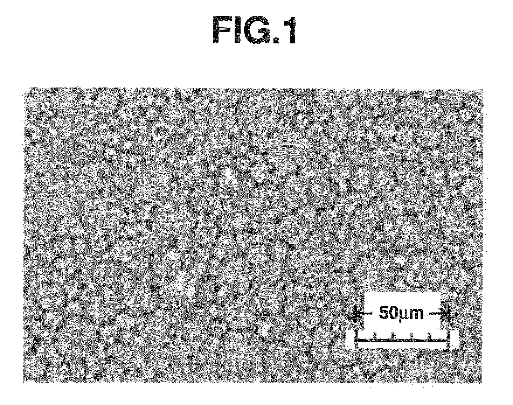

In order to confirm the particles of the water-in-silicone oil macroemulsion produced in Example 2, the composition was observed by using an optical microscope. The results are shown in FIG. 1.

FIG. 1 is a picture taken by scanning electron microscope of the water-in-silicone oil macroemulsion produced in Example 1, and as shown in FIG. 1, the particles are macroparticles having a particle size of 5 to 20 µm.

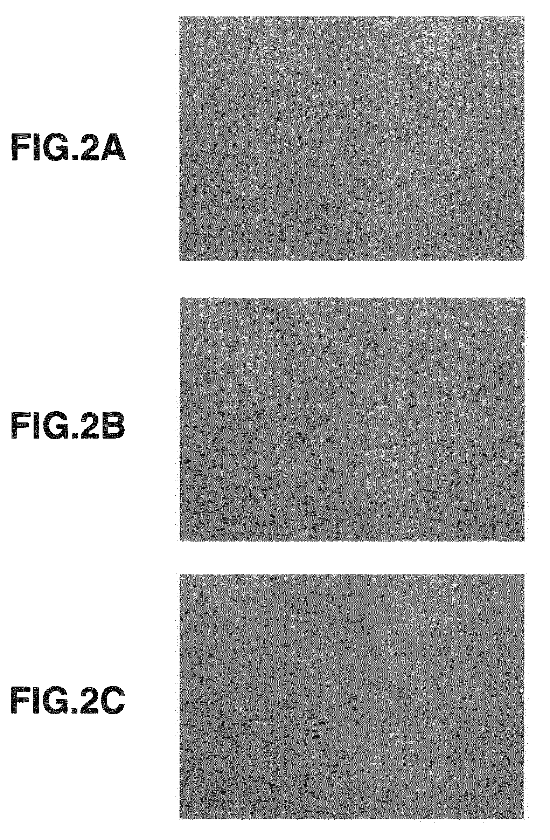

In order to evaluate the dispersion state of the water-in-silicone oil macroemulsions produced in Examples 8 and 9 and Comparative Example 4, measurement was conducted by using an optical microscope. The results are shown in FIGS. 2(a) to 2(c). FIGS. 2(a) to 2(c) are respectively pictures taken by the optical microscope of the water-in-silicone oil macroemulsion of Examples 8 and 9 and Comparative Example 4, and stable dispersion of these macroemulsions were thereby confirmed. The water-in-silicone oil macroemulsions of Comparative Example 4, however, had the problem of poor stability in the repeated freezing and thawing despite the emulsion formation.

### Test Example_2

### Evaluation of the stability

In order to confirm the stability of the water-in-silicone oil (W/S) macroemulsions produced in the Examples and Comparative Examples, the stability was evaluated by leaving the macroemulsion composition in a thermostatic tank and circulation tank. The stability was evaluated in the conditions as shown below.

**Table 4**

| Evaluation | Method |
|---|---|
| High temperature stability | Evaluated after storing in a thermostatic chamber at 45° C for 3 months |
| Room temperature stability | Evaluated after storing in a thermostatic chamber at 25° C for 3 months |
| Low temperature stability | Evaluated after storing in a thermostatic chamber at 5° C for 3 months |
| Circulation stability | Evaluated after storing in a circulating tank for 3 month |
| | 0°C (6 hr) → 15°C (6 hr) → 30°C (6 hr) → 45°C (6 hr) → 30°C (6 hr) → 15°C (6 hr) → 0°C (6 hr) |
| Freeze-thaw stability | 3 cycles of storing at -20° C for 2 weeks and storing at room temperature for 2 days |
| Evaluation | A: excellent |
| | B: good |
| | C: slight inconvenience but not practically unacceptable |
| | D: poor (discoloration, odor, separation) |

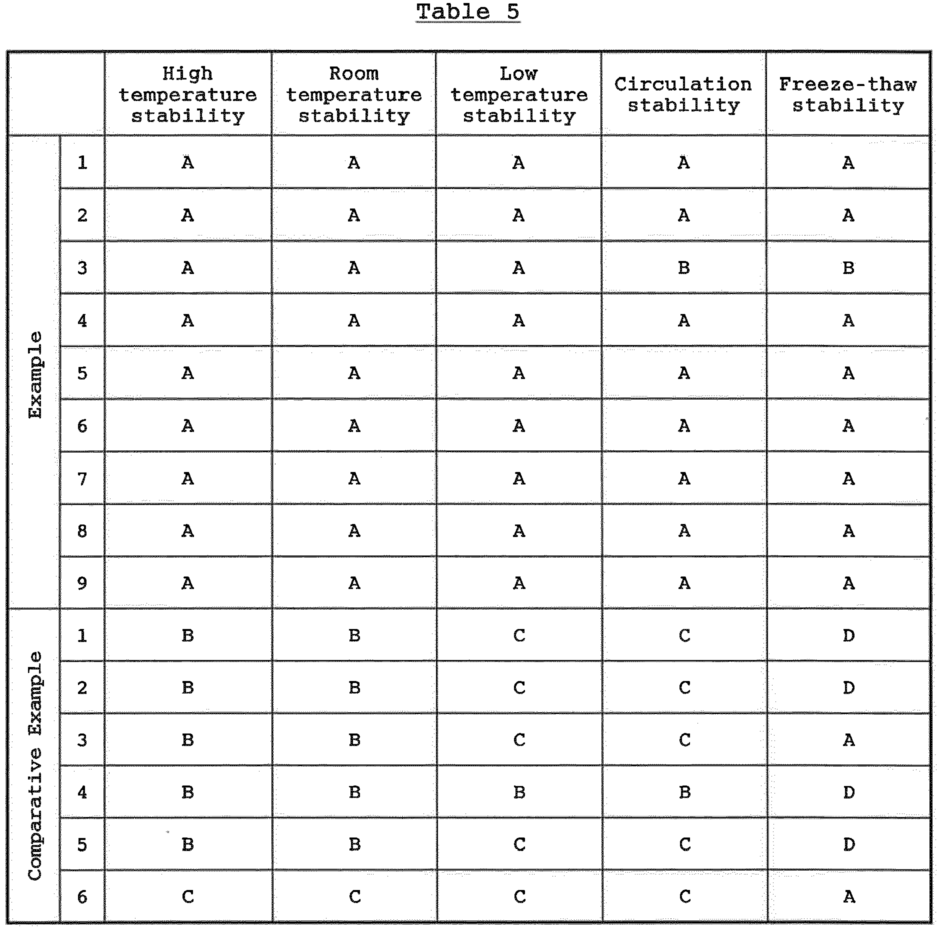

As demonstrated in Table 5, the cosmetic compositions of the present invention containing the glycerin as the freeze stabilizer and the at least one member selected from polyols and lower alcohols and/or the at least one member selected from organic acid salts and inorganic salts as a mixture of freeze stabilizers exhibit excellent stability.

In contrast, the Comparative Examples were inferior in the stability in the repeated cycles of the freezing and the thawing despite the fairly good high temperature stability, room temperature stability, low temperature stability, and circulation stability.

### Test Example 3

### Sensory test

The water-in-silicone oil macroemulsions produced in the Examples and Comparative Examples were evaluated for their waterdrop quick break effect, moist feeling, spreadability, and adhesion in a test by 20 women at 20 to 40 years old. The results are showing Table 6, below. The evaluation was conducted by the following criteria.
Poor: D
Relatively poor: C
Acceptable: B
Excellent: A

**Table 6**

| | | Waterdrop quick break effect | Moist feel | Spreadability | Adhesion |
|---|---|---|---|---|---|
| Example | 1 | A | A | A | A |
| | 2 | A | A | A | A |
| | 3 | A | A | A | A |
| | 4 | A | A | A | A |
| | 5 | A | A | A | A |
| | 6 | A | A | A | A |
| | 7 | A | A | A | A |
| | 8 | A | A | A | A |
| | 9 | A | A | A | A |
| Comparative Example | 1 | C | C | D | D |
| | 2 | C | C | D | D |
| | 3 | C | C | D | D |
| | 4 | B | B | C | C |
| | 5 | B | B | C | C |
| | 6 | D | D | D | D |

As demonstrated in Table 6, the user could feel waterdrop quick break effect of the composition of the Examples without feeling sacrifice of the spreadability, adhesion, and retention.

In contrast, the compositions of the Comparative Examples were insufficient in all of these evaluations.

### Notes

(1) In respect of numerical ranges disclosed in the present description it will of course be understood that in the normal way the technical criterion for the upper limit is different from the technical criterion for the lower limit, i.e. the upper and lower limits are intrinsically distinct proposals.
(2) For the avoidance of doubt it is confirmed that in the general description above, in the usual way the proposal of general preferences and options in respect of different features of the composition and method of making it constitutes the proposal of general combinations of those general preferences and options for the different features, insofar as they are combinable and compatible and are put forward in the same context.

## Claims

1. A water-in-silicone oil macroemulsion cosmetic composition comprising a silicone oil phase part and an aqueous phase part, wherein
the silicone oil phase part comprises a partly crosslinked emulsifiable silicone elastomer, a partly crosslinked non-emulsifiable silicone elastomer, a non-crosslinked silicone emulsifier, and a silicone oil, and
the aqueous phase part contains glycerin as a freeze stabilizer at an amount of 3 to 15% by weight of the entire composition.

2. A water-in-silicone oil macroemulsion cosmetic composition according to claim 1 further comprising at least one member selected from polyols and lower alcohols as a freeze stabilizer in the aqueous phase part at an amount of 3 to 15% by weight of the entire composition.

3. A water-in-silicone oil macroemulsion cosmetic composition according to claim 2 wherein the polyol is 1,3-butylene glycol.

4. A water-in-silicone oil macroemulsion cosmetic composition according to claim 2 or 3 wherein the lower alcohol is at least one member selected from methanol, ethanol, isopropanol, butanol, pentanol, and combinations thereof.

5. A water-in-silicone oil macroemulsion cosmetic composition according to any one of claims 1 to 4 further comprising at least one member selected from organic acid salts and inorganic salts as the freeze stabilizer in the aqueous phase part at an amount of 0.4 to 1.5% by weight of the entire composition.

6. A water-in-silicone oil macroemulsion cosmetic composition according to claim 5 wherein the organic acid salt is at least one member selected from the group consisting of sodium citrate, sodium formate, sodium acetate, and potassium acetate.

7. A water-in-silicone oil macroemulsion cosmetic composition according to claim 5 or 6 wherein the inorganic salt is at least one member selected from sodium chloride, potassium chloride, calcium chloride, magnesium chloride, and magnesium sulfate.

8. A method of making a water-in-silicone oil macroemulsion cosmetic composition according to any one of claims 1 to 7, comprising emulsifying the specified ingredients thereof.
